(19) Europäisches Patentamt

European Patent Office

Office européen des brevets

(11) **EP 0 901 454 B1**

(12) **EUROPÄISCHE PATENTSCHRIFT**

(45) Veröffentlichungstag und Bekanntmachung des Hinweises auf die Patenterteilung:
**08.11.2000 Patentblatt 2000/45**

(51) Int Cl.7: **C07C 5/48**, C07C 51/215,
C07C 51/25, C07C 53/08,
C07C 11/04, B01J 23/36

(21) Anmeldenummer: **97924952.1**

(22) Anmeldetag: **16.05.1997**

(86) Internationale Anmeldenummer:
**PCT/EP97/02522**

(87) Internationale Veröffentlichungsnummer:
**WO 97/44299 (27.11.1997 Gazette 1997/51)**

(54) **VERFAHREN ZUR HERSTELLUNG VON ESSIGSÄURE**

PROCESS FOR THE SELECTIVE PRODUCTION OF ACETIC ACID

PROCEDE DE PRODUCTION SELECTIVE D'ACIDE ACETIQUE

(84) Benannte Vertragsstaaten:
**AT BE CH DE DK ES FR GB IT LI NL SE**
Benannte Erstreckungsstaaten:
**RO SI**

(30) Priorität: **22.05.1996 DE 19620542**

(43) Veröffentlichungstag der Anmeldung:
**17.03.1999 Patentblatt 1999/11**

(73) Patentinhaber: **Celanese Chemicals Europe GmbH**
**60439 Frankfurt am Main (DE)**

(72) Erfinder:
• **BORCHERT, Holger**
**D-67278 Bockenheim a.d. Weinstrasse (DE)**
• **DINGERDISSEN, Uwe**
**D-64342 Seeheim-Jugenheim (DE)**
• **WEIGUNY, Jens**
**D-67251 Freinsheim (DE)**

(56) Entgegenhaltungen:
**EP-A- 0 407 091          EP-A- 0 480 594**

**Beschreibung**

Die vorliegende Erfindung betrifft ein Verfahren zur selektiven Herstellung von Essigsäure durch katalytische Gasphasenoxidation von Ethan und /oder Ethylen in Gegenwart eines Palladium enthaltenden Katalysators.

Die oxidative Dehydrierung von Ethan zu Ethylen in der Gasphase, bei Temperaturen > 500°C ist beispielsweise aus US-A-4 250 346, US-A-4 524 236 und US-A-4 568 790 bekannt.

So beschreibt die US-A-4 250 346 die Verwendung einer Katalysatorzusammensetzung, die die Elemente Molybdän, X und Y im Verhältnis a:b:c enthält zur Umwandlung von Ethan in Ethylen, worin

X gleich Cr, Mn, Nb, Ta, Ti, V, und/oder W ist und Y gleich Bi, Ce, Co, Cu, Fe, K, Mg, Ni, P, Pb, Sb, Si, Sn, Tl und/oder U ist und a gleich 1, b gleich 0,05 bis 1 und c gleich 0 bis 2 ist. Der Gesamtwert von c für Co, Ni und/oder Fe muß dabei weniger als 0,5 betragen.

Die Reaktion wird vorzugsweise in Anwesenheit von zugefügtem Wasser durchgeführt. Die offenbarten Katalysatoren können ebenfalls zur Oxidation von Ethan zu Essigsäure verwendet werden, wobei die Effizienz der Umwandlung zu Essigsäure bei ca. 18 %, bei einer Ethan-Umwandlung von 7,5%, liegt.

Die vorstehend genannten Schriften beschäftigen sich hauptsächlich mit der Herstellung von Ethylen, weniger mit der gezielten Herstellung von Essigsäure.

Dagegen beschreibt die EP-B-0 294 845 ein Verfahren zur selektiven Herstellung von Essigsäure aus Ethan, Ethylen oder Gemischen davon mit Sauerstoff in Gegenwart eines Katalysatorgemisches, welches mindestens A.) einen calcinierten Katalysator der Formel $Mo_xV_y$ oder $Mo_xV_yZ_y$, worin Z eines oder mehrere der Metalle Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co und Ni sein kann, und x gleich 0,5 bis 0,9 ist, y gleich 0,1 bis 0,4 ist; und z gleich 0,001 bis 1 ist und

B.) einen Ethylenhydratationskatalysator und/oder Ethylenoxidationskatalysator enthält. Bei der zweiten Katalysatorkomponente B handelt es sich insbesondere um einen Molekularsiebkatalysator oder einen Palladium enthaltenden Oxidationskatalysator.

Bei der Verwendung des beschriebenen Katalysatorgemisches und Einspeisung eines Gasgemisches bestehend aus Ethan, Sauerstoff, Stickstoff und Wasserdampf durch den Katalysator enthaltenden Reaktor beträgt die maximale Selektivität 27% bei einem Ethanumsatz von 7%.

Ein weiteres Verfahren zur Herstellung eines Produktes, das Ethylen und/oder Essigsäure enthält wird in EP-B-0 407 091 beschrieben. Hierbei werden Ethan und/oder Ethylen und ein molekularen Sauerstoff enthaltendes Gas bei erhöhter Temperatur mit einer Katalysatorzusammensetzung, die die Elemente A, X und Y enthält, in Kontakt gebracht. A ist hierbei $Mo_dRe_eW_f$, X ist Cr, Mn, Nb, Ta, Ti, V undloder W und Y ist Bi, Ce, Co, Cu, Fe, K, Mg, Ni, P, Pb, Sb, Si, Sn, Tl und/oder U. Die maximalen Selektivitäten, die bei Verwendung des beschriebenen Katalysators bei der Oxidation von Ethan zu Essigsäure erzielt werden konnten, betragen 78%. Als weitere Nebenprodukte werden Kohlendioxid, Kohlenmonoxid und Ethylen gebildet.

Keine der vorstehend aufgezählten Publikationen beschreibt jedoch die Verwendung eines Katalysators, der die Elemente Rhenium, Palladium und Molybdän enthält, zur selektiven Oxidation von Ethan und/oder Ethylen zu Essigsäure. Ferner sind die bis jetzt im Stand der Technik erzielten Selektivitäten für die Oxidation zu Essigsäure noch nicht befriedigend.

Es besteht daher die Aufgabe ein Verfahren zur Verfügung zu stellen, das es erlaubt Ethan und/oder Ethylen in einfacher Weise, gezielt und mit hoher Selektivität zu Essigsäure zu oxidieren.

Überraschend wurde nun gefunden, daß es möglich ist, bei Verwendung eines Katalysators, der die Elemente Molybdän, Rhenium und Palladium und eines oder mehrere Elemente aus der Gruppe Chrom, Mangan, Niob, Tantal, Titan, Vanadium und/oder Wolfram enthält, Ethan und/oder Ethylen unter relativ milden Bedingungen, in einfacher Weise mit hoher Selektivität zu Essigsäure zu oxidieren.

Die vorliegende Erfindung betrifft somit ein Verfahren zur selektiven Herstellung von Essigsäure aus gasförmigem Ethan, Ethylen oder Gemischen davon sowie Sauerstoff bei erhöhter Temperatur an einem Katalysator, dadurch gekennzeichnet, daß die gasförmige Einspeisung mit einem Katalysator zusammengebracht wird, der die Elemente Mo, Pd, Re, X und Y in den Grammatomverhältnissen a:b:c:d:e in Kombination mit Sauerstoff enthält

$$Mo_aPd_bRe_cX_dY_e \qquad\qquad\qquad (I)$$

und die Symbole X, Y folgende Bedeutung haben:

X = Cr, Mn, Nb, B, Ta, Ti, V und/oder W, insbesondere Nb, V und W
Y = Bi, Ce, Co, Cu, Te, Fe, Li, K, Na, Rb,Be, Mg, Ca, Sr, Ba, Ni, P, Pb, Sb, Si, Sn, Tl und/oder U, insbesondere Ca, Sb, Te und Li.

Die Indizes a, b, c, d und e stehen für die Grammatomverhältnisse der entsprechenden Elemente, wobei a = 1, b > 0, c > 0, d = 0,05 bis 2 und e = 0 bis 3 ist.

Sofern X und Y für mehrere verschiedene Elemente stehen, können die Indizes d und e ebenfalls mehrere unterschiedliche Werte annehmen.

Weiterhin betrifft die vorliegende Erfindung einen Katalysator zur selektiven Herstellung von Essigsäure enthaltend die Elemente Mo, Pd, Re, X und Y in den Grammatomverhältnissen a:b:c:d:e in Kombination mit Sauerstoff.

Die Grammatomverhältnisse a:b:c:d:e liegen vorzugsweise in folgenden Bereichen:

a = 1; b = 0,0001 bis 0,5; c = 0,25 bis 1,0; d = 0,1 bis 1,0;

e = 0 bis 1,0.

Palladiumgehalte im Katalysator, die über der angegebenen Obergrenze liegen führen bei dem erfindungsgemäßen Verfahren zu einer Begünstigung der Kohlendioxidbildung. Ferner werden höhere Palladiumgehalte allgemein auch deshalb vermieden, da sie den Katalysator unnötig verteuern. Dagegen wird bei Palladiumgehalten unterhalb des angegebenen Grenzwertes eine Bevorzugung der Ethylenbildung beobachtet.

Rheniumgehalte, die unterhalb des angegeben Grenzwertes liegen führen ebenfalls zu einer bevorzugten Bildung von Ethylen auf Kosten der Selektivität zu Essigsäure. Rheniumgehalte, die höher als der angegebene Grenzwert sind, bewirken dagegen keine weitere Verbesserung der katalytischen Eigenschaften und würden daher auch hier den Katalysator nur unnötig verteuern.

Vorzugsweise enthält der erfindungsgemäß verwendete Katalysator außer den Elementen Molybdän, Palladium und Rhenium noch Vanadium, Niob, Antimon und Kalzium in Kombination mit Sauerstoff. Die Grammatomverhältnisse $a{:}b{:}c{:}d^1{:}d^2{:}e^1{:}e^2$ der Elemente Mo:Pd:Re:V:Nb:Sb:Ca sind vorzugsweise wie folgt:

a (Mo)=1;      b (Pd)= 0,0001 bis 0,5, insbesondere 0,001 bis 0,05;

c (Re)=0,25 bis 1,0;      $d^1$ (V)= 0,2 bis 1,0;      $d^2$ (Nb)= 0,1 bis 0,5;

$e^1$ (Sb)= 0 bis 0,5;      $e^2$(Ca)= 0 bis 0,2;

Beispiele für derartige im erfindungsgemäßen Verfahren bevorzugt eingesetzte Katalysatorzusammensetzungen sind:

$$Mo_{1,0}Pd_{0,01}Re_{0,7}V_{0,7}Nb_{0,2}Sb_{0,1}Ca_{0,05}$$
$$Mo_{1,0}Pd_{0,02}Re_{0,7}V_{0,7}Nb_{0,2}Sb_{0,1}Ca_{0,05}$$
$$Mo_{1,0}Pd_{0,02}Re_{0,5}V_{0,5}Nb_{0,5}Sb_{0,1}$$
$$Mo_{1,0}Pd_{0,02}Re_{0,7}V_{0,5}Te_{0,5}$$
$$Mo_{1,0}Pd_{0,02}Re_{0,7}V_{0,7}Nb_{0,2}Sb_{0,1}Ca_{0,05}$$
$$Mo_{1,0}Pd_{0,02}Re_{0,7}W_{0,2}V_{0,7}Nb_{0,2}Sb_{0,1}$$

Die erfindungsgemäß verwendeten Katalysatoren können nach den herkömmlichen Verfahren hergestellt werden. Hierzu geht man von einer Aufschlämmung, insbesondere einer wässrigen Lösung, die die einzelnen Ausgangskomponenten der Elemente entsprechend ihrer Anteile enthält, aus.

Die Ausgangsmaterialien der Einzelkomponenten zur Herstellung des erfindungsgemäßen Katalysators sind neben den Oxiden vorzugsweise in Wasser lösliche Substanzen wie Ammoiumsalze, Nitrate, Sulfate, Halogenide, Hydroxide und Salze organischer Säuren, die durch Erwärmung in die entsprechenden Oxide umgewandelt werden können. Zur Vermischung der Komponenten werden wäßrige Lösungen oder Suspensionen der Metallsalze hergestellt und vermischt.

Bei Molybdän empfiehlt es sich aufgrund der kommerziellen Verfügbarkeit als Ausgangsverbindungen die entsprechenden Molybdate, wie z.B. Ammoniummolybdat, einzusetzen.

Als Palladiumverbindungen kommen beispielsweise Palladium(II)-chlorid, Palladium(II)-sulfat, Palladium(II)-tetraminnitrat, Palladium(II)-nitrat sowie Palladium(II)-acetonylacetonat in Frage.

Im Falle des Rheniums können zum Beispiel Perrheniumsäure, Ammoniumperrhenat sowie Rhenium(III)- und Rhenium(V)-chloridm, um nur einige zu nennen, als Ausgangsverbindung eingesetzt werden.

Die erhaltene Reaktionsmischung wird dann 5 Minuten bis 5 Stunden bei 50 bis 100 °C gerührt. Anschließend wird das Wasser entfernt und der verbleibende Katalysator bei einer Temperatur von 50 bis 150°C, insbesondere 80 bis 120°C getrocknet.

Für den Fall, daß der erhaltene Katalysator anschließend noch einem Kalzinierungsprozeß unterworfen wird, empfiehlt es sich den getrockneten und pulverisierten Katalysator bei einer Temperatur im Bereich von 100°C bis 800°C, insbesondere 200 bis 500°C in Gegenwart von Stickstoff, Sauerstoff oder eines sauerstoffhaltigen Gases zu kalzinieren. Die Zeitdauer beträgt 2 bis 24 Stunden.

Der Katalysator kann ohne ein entsprechendes Trägermaterial eingesetzt werden oder mit einem solchen gemischt oder auf ein solches aufgebracht werden. Geeignet sind übliche Trägermaterialien, wie z.B. poröses Siliziumdioxid, geglühtes Siliziumdioxid, Kieselgur, Kieselgel, poröses oder nicht poröses Aluminiumoxid, Titandioxid, Zirkoniumdioxid, Thoriumdioxid, Lanthanoxid, Magnesiumoxid, Calciumoxid, Bariumoxid, Zinnoxid, Cerdioxid, Zinkoxid, Boroxid, Bornitrid, Borcarbid, Borphosphat, Zirkoniumphosphat, Aluminiumsilikat, Siliziumnitrid oder Siliziumcarbid aber auch Glas- oder Metallnetze.

Bevorzugte Trägermaterialien haben eine Oberfläche von weniger als 100 $m^2$/g. Bevorzugte Trägermaterialien sind Siliziumdioxid und Aluminiumoxid mit geringer spezifischer Oberfläche. Der Katalysator kann nach der Formgebung als regelmäßig oder unregelmäßig geformter Trägerkörper oder aber in Pulverform als heterogener Oxidationskatalysator eingesetzt werden.

Die Reaktion kann in der Wirbelschicht oder in einem Festbettreaktor durchgeführt werden. Für den Einsatz in einer Wirbelschicht wird der Katalysator auf eine Korngröße im Bereich von 10 bis 200 µm gemahlen.

Die gasförmige Einspeisung enthält Ethan und/oder Ethylen, welche als reine Gase oder in Mischung mit einem oder mehreren anderen Gasen dem Reaktor zugeführt werden. Als solche zusätzlichen oder Trägergase kommen beispielsweise Stickstoff, Methan, Kohlenmonoxid, Kohlendioxid, Luft und/oder Wasserdampf in Frage. Das molekularen Sauerstoff enthaltende Gas kann Luft oder ein an molekularen Sauerstoff reicheres oder ärmeres Gas als Luft, z.B. Sauerstoff, sein. Bevorzugt ist die Zugabe von Wasserdampf zum Ethan und molekularen Sauerstoff enthaltenden Gas, da hierdurch die Selektivität zu Essigsäure begünstigt wird. Der Anteil des Wasserdampfes liegt im Bereich von 5 bis 30 Vol%, vorzugsweise 10 bis 20 Vol%. Niedrigere Wasserdampfgehalte führen zu einem Selektivitätsverlust hinsichtlich der Essigsäurebildung, während höhere Wasserdampfkonzentrationen die Aufarbeitung der anfallenden wässrigen Essigsäure aus verfahrenstechnischen Gründen unnötig verteuern würden. Die Zugabe von Sauerstoff oder des molekularen Sauerstoff enthaltenen Gases richtet sich nach den Explosionsgrenzen unter Reaktionsbedingungen. Höhere Sauerstoffgehalte sind bevorzugt, da der erreichbare Ethanumsatz und somit die Ausbeute an Essigsäure höher ist. Die obere Sauerstoffkonzentration ist dagegen durch die Explosionsgrenzen limitiert. Das Verhältnis von Ethan zu Sauerstoff liegt günstigerweise im Bereich zwischen 1:1 und 10:1, vorzugsweise 2:1 und 8:1.

Die Reaktion wird bei Temperaturen zwischen 200 und 500°C, bevorzugt 200 bis 400°C durchgeführt. Der Druck kann atmosphärisch oder superatmosphärisch sein, z.B. im Bereich zwischen 1 und 50 bar, bevorzugt 1 bis 30 bar.

Die Reaktion kann in einem Festbett- oder Wirbelschichtreaktor durchgeführt werden. Zweckmäßigerweise wird Ethan zunächst mit den inerten Gasen wie Stickstoff oder Wasserdampf gemischt, bevor Sauerstoff oder das molekularen Sauerstoff enthaltende Gas zugeführt wird. Die vermischten Gase werden bevorzugt in einer Vorheizzone auf die Reaktionstemperatur vorgeheizt, bevor das Gasgemisch mit dem Katalysator in Kontakt gebracht wird. Aus dem Reaktorabgas wird Essigsäure durch Kondensation abgetrennt. Die übrigen Gase werden an den Reaktoreingang zurückgeführt, wo Sauerstoff oder das molekularen Sauerstoff enthaltende Gas sowie Ethan und/oder Ethylen zudosiert wird.

Bei Verwendung des erfindungsgemäßen Katalysators liegt die Selektivität bei der Oxidation von Ethan und/oder Ethylen zu Essigsäure bei >75 Mol%, vorzugsweise > 80 Mol%, insbesondere > 85 Mol%, bei einem Ethanumsatz von >3%, vorzugsweise >4%, insbesondere > 5%, so daß mit dem erfindungsgemäßen Verfahren im Vergleich mit dem Stand der Technik eine Erhöhung der Essigsäureausbeuten, bei gleichzeitiger Verminderung des Anfalls von ungewünschten Nebenprodukten, auf einfache Weise erzielt werden kann.

Beispiele

Die in den Beispielen angegebene Katalysatorzusammensetzung sind in relativen Atomverhältnissen angegeben.

Katalysatorpräparation:

Katalysator (I):

Ein Katalysator enthaltend die Elemente in nachfolgend aufgeführter Zusammensetzung (in Kombination mit Sauerstoff) wurde hergestellt:
$Mo_{1,00}Re_{0,67}V_{0,70}Nb_{0,19}Sb_{0,08}Ca_{0,05}Pd_{0,01}$

Lösung 1:

10,0 g Ammoniumperrhenat, 0,12 g Palladiumacetat und 9,7 g Ammoniummolybdat in 50 ml Wasser.

Lösung 2:

4,5 g Ammoniummetavanadat in 50 ml Wasser.

Lösung 3:

6,5 g Nioboxalat, 1,34 g Antimonoxalat, 0,58 g Calciumnitrat in 180 ml Wasser.

Die Lösungen werden separat bei 70°C für 15 Minuten gerührt. Dann wird die dritte Lösung zur zweiten hinzugegeben. Die vereinigten Mischungen werden bei 70°C für 15 Minuten gerührt bevor diese zur ersten gegeben werden. Die erhaltene Mischung wird bei 70°C für 15 Minuten gerührt. Anschließend wird das Wasser auf einer heißen Platte entfernt bis eine dicke Paste entsteht. Diese wird bei 120°C über Nacht getrocknet. Der Feststoff wird zerstoßen (Siebfraktion: 0,35 bis 2 mm) und anschließend in statischer Luft bei 300°C für 5 Stunden calciniert. Der Katalysator wird hiernach gesiebt, um eine Siebfraktion zwischen 0,35 und 1 mm zu erhalten.

Katalysator (II): Ein Katalysator enthaltend die Elemente in nachfolgend aufgeführter Zusammensetzung (in Kombination mit Sauerstoff) wurde hergestellt:

$Mo_{1,00}Re_{0,67}V_{0,70}Nb_{0,19}Sb_{0,08}Ca_{0,05}Pd_{0,02}$

Die Herstellung erfolgte wie in Katalysatorbeispiel (I) beschrieben, mit der Änderung, daß statt 0,12 g Palladiumacetat 0,24 g eingesetzt wurden.

Vergleichsbeispiel

Katalysator (III):

Zum Vergleich wurde ein Katalysator entsprechend EP 0 407 091 mit folgender Zusammensetzung hergestellt:

$Mo_{1,00}Re_{0,67}V_{0,70}Nb_{0,19}Sb_{0,08}Ca_{0,05}$

Die Herstellung erfolgte wie in Katalysatorbeispiel (I) beschrieben, mit der Änderung, daß kein Palladiumacetat eingesetzt wurde.

Der in EP-B-0 407 091, Tabelle 2, angegebene Umsatz von 14,3 % kann selbst bei vollständiger Konversion des Sauerstoffs aus stöchiometrischen Gründen nicht erreicht werden. Bei den angegebenen Selektivitäten und der Zusammensetzung des Eingangsgases, kann der Umsatz maximal 5,9% betragen. Bei dieser Berechnung wurde vorausgesetzt, daß neben Essigsäure und Ethylen nur Kohlenmonoxid gebildet wird. Für den Fall, daß statt Kohlenmonoxid auch Kohlendioxid gebildet wird, liegt der maximal erreichbare Ethanumsatz bei nur 5,5%. Es ist zu vermuten, daß auf Grund der Versuchsdurchführung Ethan in der dem Reaktor nachgeschalteten Kühlfalle auskondensiert wurde, was zur falschen Berechnung eines zu hohen Umsatz führte. Um die katalytischen Eigenschaften dieses Katalysators mit dem erfindungsgemäßen Katalysators zu vergleichen, wurden beide Katalysatoren unter gleichen Reaktionsbedingungen getestet (siehe Vergleichsbeispiel).

Methode zur Katalysatoraustestung

10 ml des Katalysators wurde in einen Stahlreaktor mit 10 mm Innendurchmesser geladen. Der Katalysator wurde unter einem Luftstrom auf 250°C aufgeheizt. Anschließend wurde der Druck mittels eines Vordruckreglers eingestellt. Das gewünschte Ethan:Sauerstoff:Stickstoff-Gemisch wurde mit Wasser in eine Verdampferzone eindosiert, wo Wasser verdampfte und mit den Gasen vermischt wurde. Die Reaktionstemperatur wurde mit einem Thermoelement in der Katalysatorschüttung gemessen. Das Reaktionsgas wurde on-line gaschromatographisch analysiert.

In den Beispielen sind die folgende Begriffe definiert als:

$$Ethanumsatz\ (\%) =$$

$$100x([CO]/2+[CO_2]/2+[C_2H_4]+[CH_3COOH])/([CO]/2+[CO_2]/2+[C_2H_4]+[C_2H_6]+\ [CH_3COOH])$$

$$Ethylenselektivität\ (\%) =$$

$$100x([C_2H_4])/([CO]/_2+[CO_2]/2+[C_2H_4]+[CH_3COOH])$$

$$Essigsäureselektiviät\ (\%) =$$

$$100x([CH_3COOH])/([CO]/_2+[CO_2]/2+[C_2H_4]+[CH_3COOH])$$

worin [ ] = Konzentrationen in mol% und

[$C_2H_6$] = Konzentration des nicht umgesetzten Ethans bedeutet.

Die Verweilzeit ist definiert als:

t (s) = Schüttvolumen des Katalysators (ml) / Volumenstrom des Gases durch den Reaktor bezogen auf die Reaktionsbedingungen (ml/s)

Reaktionsdurchführung:

Die Reaktion wurde bei 280°C und 15 bar durchgeführt. Das Reaktoreingangsgas bestand aus 40 Vol% Ethan, 8 Vol% Sauerstoff, 32 Vol% Stickstoff und 20 Vol% Wasserdampf. Die Ergebnisse sind in nachfolgender Tabelle zusammengefaßt.

| Katalysator | Verweilzeit (s) | Ethanumsatz (%) | Essigsäureselektivität (%) | Ethylenselektivität (%) | CO + $CO_2$ Selektivität (%) |
|---|---|---|---|---|---|
| (I) | 30 | 3 | 91 | 0 | 9 |
| (II) | 30 | 4 | 91 | 0 | 9 |
| (II) | 60 | 8 | 90 | 2 | 8 |
| (III) | 30 | 5 | 61 | 29 | 10 |

Im Vergleich zu Katalysator (III) werden mit den Katalysatoren (I) und (II) höhere Selektivitäten zu Essigsäure erhalten, ohne daß die CO+$CO_2$-Selektivitäten verstärkt werden. Dies führt zu einer verbesserten Essigsäureausbeute bezogen auf die eingesetzte Katalysatormenge und den zugeführten Ethanstrom.

**Patentansprüche**

1. Verfahren zur selektiven Herstellung von Essigsäure aus gasförmigem Ethan, Ethylen oder Gemischen davon sowie Sauerstoff bei erhöhter Temperatur an einem Katalysator, dadurch gekennzeichnet, daß die gasförmige Einspeisung mit einem Katalysator zusammengebracht wird, der die Elemente Mo, Pd, Re, X und Y in den Grammatomverhältnissen a:b:c:d:e in Kombination mit Sauerstoff enthält

$$Mo_aPd_bRe_cX_dY_e \qquad\qquad (I)$$

wobei die Symbole X, Y folgende Bedeutung haben:

X = Cr, Mn, Nb, B, Ta, Ti, V und/oder W
Y = Bi, Ce, Co, Cu, Te, Fe, Li, K, Na, Rb,Be, Mg, Ca, Sr, Ba, Ni, P, Pb, Sb, Si, Sn, Tl und/oder U;
die Indizes a, b, c, d und e stehen für die Grammatomverhältnisse der entsprechenden Elemente, wobei
a = 1, b > 0, c > 0, d = 0,05 bis 2, e = 0 bis 3 ist.

2. Verfahren nach Anspruch 1, dadurch gekennzeichnet, daß die Reaktionstemperatur im Bereich von 200 bis 500°C liegt.

3. Verfahren nach mindestens einem der Ansprüche 1 bis 2, dadurch gekennzeichnet, daß der Druck im Reaktor im Bereich von 1 bis 50 bar liegt.

4. Verfahren nach mindestens einem der Ansprüche 1 bis 3, dadurch gekennzeichnet, daß b = 0,0001 bis 0,5 ist.

5. Verfahren nach mindestens einem der Ansprüche 1 bis 4, dadurch gekennzeichnet, daß dem Reaktor das Ethan gemischt mit mindestens einem weiteren Gas, insbesondere Stickstoff, Methan, Kohlendioxid, Kohlenmonoxid, Ethylen und/oder Wasserdampf zugeführt wird.

6. Verfahren nach mindestens einem der Ansprüche 1 bis 5, dadurch gekennzeichnet, daß der Katalysator mindestens eine der folgenden Zusammensetzungen in Kombination mit Sauerstoff enthält.

$Mo_{1,0}Pd_{0,01}Re_{0,7}V_{0,7}Nb_{0,2}Sb_{0,1}Ca_{0,05}$
$Mo_{1,0}Pd_{0,02}Re_{0,7}V_{0,7}Nb_{0,2}Sb_{0,1}Ca_{0,05}$
$Mo_{1,0}Pd_{0,02}Re_{0,5}V_{0,5}Nb_{0,5}Sb_{0,1}$
$Mo_{1,0}Pd_{0,02}Re_{0,7}V_{0,5}Te_{0,5}$
$Mo_{1,0}Pd_{0,02}Re_{0,7}V_{0,7}Nb_{0,2}Sb_{0,1}Ca_{0,05}$
$Mo_{1,0}Pd_{0,02}Re_{0,7}W_{0,2}V_{0,7}Nb_{0,2}Sb_{0,1}$

wobei die Selektivität der Oxidationsreaktion von Ethan und/oder Ethylen zu Essigsäure bei > 75 Mol%, bei einem Ethanumsatz von > 3%, liegt.

7. Verfahren nach mindestens einem der Ansprüche 1 bis 6, dadurch gekennzeichnet, daß der Katalysator mit einem Trägermaterial gemischt oder auf einem Trägermaterial fixiert ist.

8. Katalysator zur selektiven Oxidation von Ethan und/oder Ethylen zu Essigsäure enthaltend die Elemente Mo, Pd, Re, X und Y in den Grammatomverhältnissen a:b:c:d:e in Kombination mit Sauerstoff

$$Mo_aPd_bRe_cX_dY_e \qquad (I)$$

wobei die Symbole X, Y folgende Bedeutung haben:

X = Cr, Mn, Nb, B, Ta, Ti, V und/oder W
Y = Bi, Ce, Co, Cu, Te, Fe, Li, K, Na, Rb, Be, Mg, Ca, Sr, Ba, Ni, P, Pb, Sb, Si, Sn, Tl und/oder U;
die Indizes a, b, c, d und e stehen für die Grammatomverhältnisse der entsprechenden Elemente, wobei
a = 1, b > 0, c > 0, d = 0,05 bis 2, e = 0 bis 3 ist.

## Claims

1. A process for the selective preparation of acetic acid from gaseous ethane, ethylene or mixtures thereof plus oxygen at elevated temperature in the presence of a catalyst, which comprises bringing the gaseous feed into contact with a catayst comprising the elements Mo, Pd, Re, X and Y in gram atoms ratios a:b:c:d:e in combination with oxygen

$$Mo_aPd_bRe_cX_dY_e \qquad (I)$$

where the symbols X, Y have the following meanings:

X = Cr, Mn, Nb, B, Ta, Ti, V and W
Y = Bi, Ce, Co, Cu, Te, Fe, Li, K, Na, Rb, Be, Mg, Ca, Sr, Ba, Ni, P, Pb, Sb, Si, Sn, Tl and/or U;
the indices a, b, c, d and e are the gram atom ratios of the corresponding elements, where
a = 1, b > 0, c > 0, d = 0.05-2, e = 0-3.

2. The process as claimed in claim 1, wherein the reaction temperature is in the range from 200 to 500°C.

3. The process as claimed in at least one of claims 1 to 2, wherein the pressure in the reactor is in the range from 1 to 50 bar.

4. The process as claimed in at least one of claims 1 to 3, wherein b = 0.0001 to 0.5.

5. The process as claimed in at least one of claims 1 to 4, wherein the ethane mixed with at least one further gas, in particular nitrogen, methane, carbon monoxide, carbon dioxide, ethylene and/or water vapor, is fed to the reactor.

6. The process as claimed in at least one of claims 1 to 5, wherein the catalyst comprises at least one of the following compositions in combination with oxygen:

$Mo_{1.0}Pd_{0.01}Re_{0.7}V_{0.7}Nb_{0.2}Sb_{0.1}Ca_{0.05}$
$Mo_{1.0}Pd_{0.02}Re_{0.7}V_{0.7}Nb_{0.2}Sb_{0.1}Ca_{0.05}$
$Mo_{1.0}Pd_{0.02}Re_{0.5}V_{0.5}Nb_{0.5}Sb_{0.1}$
$Mo_{1.0}Pd_{0.02}Re_{0.7}V_{0.5}Te_{0.5}$
$Mo_{1.0}Pd_{0.02}Re_{0.7}V_{0.7}Nb_{0.2}Sb_{0.1}Ca_{0.05}$
$Mo_{1.0}Pd_{0.02}Re_{0.7}W_{0.2}V_{0.7}Nb_{0.2}Sb_{0.1}$

where the selectivity of the oxidation reaction of ethane and/or ethylene to form acetic acid is >75 mol%, at an ethane conversion of >3%.

7. The process as claimed in at least one of claims 1 to 6, wherein the catalyst is mixed with a support material or is fixed on a support material.

8. A catalyst for the selective oxidation of ethane and/or ethylene to form acetic acid, comprising the elements Mo, Pd, Re, X and Y in the gram atom ratios a:b:c:d:e in combination with oxygen

$$Mo_aPd_bRe_cX_dY_e \qquad (I)$$

where the symbols X, Y have the following meanings:

X = Cr, Mn, Nb, B, Ta, Ti, V and/or W
Y = Bi, Ce, Co, Cu, Te, Fe, Li, K, Na, Rb, Be, Mg, Ca, Sr, Ba, Ni, P, Pb, Sb, Si, Sn, Tl and/or U;
the indices a, b, c, d and e are the gram atom ratios of the corresponding elements, where
a = 1, b > 0, c > 0, d = 0.05-2, e = 0-3.

## Revendications

1. Procédé de préparation sélective de l'acide acétique à partir d'éthane gazeux, d'éthylène gazeux ou de mélanges de ceux-ci ainsi que d'oxygène à haute température sur un catalyseur, caractérisé en ce que la charge gazeuse est mise en contact avec un catalyseur qui contient les éléments Mo, Pd, Re, X et Y dans les proportions en atomes-grammes a:b:c:d:e en combinaison avec de l'oxygène

$$Mo_aPd_bRe_cX_dY_e \qquad (I)$$

et les symboles X. Y ont la signification suivante :

X = Cr, Mn, Nb, B, Ta, Ti, V et/ou W,
Y = Bi, Ce, Co, Cu, Te, Fe, Li, K, Na, Rb, Be, Mg, Ca, Sr, Ba, Ni, P, Pb, Sb, Si, Sn. Tl et/ou U.
les indices a, b, c, d et e représentent les proportions en atomes-grammes des éléments correspondants, avec
a = 1, b>0, c>0, d = 0,05 à 2, e = 0 à 3.

2. Procédé selon la revendication 1, caractérisé en ce que la température de réaction est située dans le domaine de 200 à 500°C.

3. Procédé selon au moins l'une des revendications 1 et 2, caractérisé en ce que la pression dans le réacteur est située dans le domaine de 1 à 50 bars.

4. Procédé selon au moins l'une des revendications 1 à 3, caractérisé en ce que b = 0,0001 à 0.5.

5. Procédé selon au moins l'une des revendications 1 à 4, caractérisé en ce que l'éthane est introduit dans le réacteur en mélange avec au moins un autre gaz, en particulier l'azote, le méthane, le dioxyde de carbone, le monoxyde de carbone, l'éthylène et/ou la vapeur d'eau.

6. Procédé selon au moins l'une des revendications 1 à 5, caractérisé en ce que le catalyseur contient au moins l'une des compositions suivantes en combinaison avec de l'oxygène

$Mo_{1,0}Pd_{0,01}Re_{0,7}V_{0,7}Nb_{0,2}Sb_{0,1}Ca_{0,05}$
$Mo_{1,0}Pd_{0,02}Re_{0,7}V_{0,7}Nb_{0,2}Sb_{0,1}Ca_{0,05}$
$Mo_{1,0}Pd_{0,02}Re_{0,5}V_{0,5}Nb_{0,5}Sb_{0,1}$
$Mo_{1,0}Pd_{0,02}Re_{0,7}V_{0,5}Te_{0,5}$
$Mo_{1,0}Pd_{0,02}Re_{0,7}V_{0,7}Nb_{0,2}Sb_{0,1}Ca_{0,05}$
$Mo_{1,0}Pd_{0,02}Re_{0,7}W_{0,2}V_{0,7}Nb_{0,}Sb_{0,1}$

où la sélectivité de la réaction d'oxydation de l'éthane et/ou de l'éthylène en acide acétique est supérieure à 75 mol % pour une conversion de l'éthane supérieure à 3 %.

7.  Procédé selon au moins l'une des revendications 1 à 6, caractérisé en ce que le catalyseur est mélangé avec un support ou est fixé sur un support.

8.  Catalyseur pour l'oxydation sélective de l'éthane et/ou de l'éthylène en acide acétique contenant les éléments Mo, Pd, Re, X et Y dans les proportions en atomes-grammes a:b:c:d:e en combinaison avec de l'oxygène

$$Mo_3Pd_bRe_cX_dY_e \qquad (I)$$

où les symboles X, Y ont la signification suivante :

X = Cr, Mn, Nb, B, Ta, Ti, V et/ou W
Y = Bi, Ce, Co, Cu, Te, Fe, Li, K, Na, Rb, Be, Mg, Ca, Sr, Ba, Ni, P, Pb, Sb, Si, Sn, Tl et/ou U ;
les indices a, b, c, d et e représentent les proportions en atomes-grammes des éléments correspondants, avec a = 1, b>0, c>0, d = 0.05 à 2, e = 0 à 3.